Europäisches Patentamt

European Patent Office

Office européen des brevets

(11) Numéro de publication: **0 497 072 A1**

## DEMANDE DE BREVET EUROPEEN

(21) Numéro de dépôt: **91440014.8**

(22) Date de dépôt: **22.02.91**

(51) Int. Cl.⁵: **A61F 13/15**

(30) Priorité: **30.01.91 FR 9101290**

(43) Date de publication de la demande:
**05.08.92 Bulletin 92/32**

(84) Etats contractants désignés:
**AT BE CH DE DK ES GB GR IT LI LU NL SE**

(71) Demandeur: **CELATOSE Société anonyme dite:**
**47, Rue de Bradford**
**F-59200 Tourcoing (Nord)(FR)**

(72) Inventeur: **Leroy, Daniel**
**104 boulevard d'Armentières**
**F-59100 Roubaix(FR)**
Inventeur: **Pringuer, Jean-Pierre**
**18 rue de Croix**
**F-59290 Wasquehal(FR)**

(74) Mandataire: **Kügele, Bernhard**
**NOVAPAT FRANCE 63 bis, boulevard**
**Bessières**
**F-75017 Paris(FR)**

(54) **Couche-culotte et son procédé de fabrication.**

(57) L'invention est relative à une couche culotte, qui trouvera notamment son application dans le domaine de l'hygiène infantile et/ou médicale, ainsi qu'à son procédé de fabrication.

La couche culotte est formée d'un film extérieur étanche (1), d'un matelas central de matériau absorbant (2) éventuellement additionné de produit superabsorbant (3), d'un tissue (4) et d'une feuille de confort (5).

Selon l'invention, du produit superabsorbant (14) est directement fixé sur la face interne du tissue (4) par collage dans des régions privilégiées à haut pouvoir absorbant.

Selon le procédé de fabrication de l'invention, on encolle la bande de tissue pour créer des zones adhésives. On projette régulièrement sur la bande de tissue le produit superabsorbant en poudre par exemple à l'aide d'un accélérateur de particules alimenté en débit constant. On récupère la quantité de produit superabsorbant résiduelle non fixée sur la bande de tissue par les zones adhésives.

FIG.1

EP 0 497 072 A1

L'invention est relative à une couche culotte ainsi qu'à son procédé de fabrication qui trouvera notamment son application dans le domaine de l'hygiène infantile et/ou de l'incontinence.

A l'origine, le change jetable a été créé pour offrir à la clientèle une plus grande commodité d'utilisation que le lange textile traditionnel réutilisable qui nécessitait un nettoyage périodique entre chaque emploi. Pour réussir commercialement, le produit devait être économique et c'est ce critère majeur qui avait été retenu par les fabricants pour orienter le développement des premiers produits.

Il est souvent difficile d'allier économie et qualité, or la clientèle s'est montrée exigeante obligeant les fabricants à toujours apporter plus de sophistication dans l'élaboration des couches culottes.

A ce niveau, un pas décisif a été franchi lorsque la notion de couche culotte avec adhésifs repositionnables est intervenue. En effet, les progrès accomplis dans le domaine des adhésifs ont fait qu'il est possible actuellement d'utiliser à plusieurs reprises la fixation et ainsi de défaire et remettre en place la couche culotte sur l'enfant. La durée d'utilisation étant ainsi allongée parallèlement il a été possible de consacrer une plus forte somme à sa fabrication qui pouvait s'amortir plus facilement.

L'étanchéïté au niveau des jambes et de la ceinture a été améliorée grâce à l'utilisation de techniques et d'accessoires plus soignés encore que la limite de réutilisation de la couche culotte était imposée par sa capacité d'absorption et de rétention des liquides.

En effet, sous peine d'inconfort, l'épaisseur de matériau absorbant utilisée pour la confection de la couche culotte ne peut dépasser un certain seuil. La chimie a permis d'apporter une solution satisfaisante à ce problème avec la mise au point d'un additif à fort pouvoir absorbant incorporé dans le matelas cellulosique central.

Ce produit superabsorbant est un polyacrylate de potassium ou de sodium obtenu par polymérisation d'acide acrylique avec un carbonate de soude. Cet additif chimique a une capacité d'absorption en eau de plusieurs dizaines de fois son poids et se présente sous forme d'une poudre très fine.

Les performances de ce produit superabsorbant sont extrêmement appréciables, cependant, certaines précautions d'utilisation doivent être prises. En effet, une trop forte concentration de ce produit provoque l'effet inverse à celui recherché c'est-à-dire un blocage à l'absorption des liquides dûe à la formation d'une barrière étanche appelée "gel blocking". Il faut également souligner qu'il s'agit d'un produit chimique coûteux qu'il faut utiliser avec rentabilité.

Les plus récents développements en la matière ont permis de mettre au point des couches culottes spécialisées. Celles-ci comportent une première incorporation de produits superabsorbants placés dans le fond du matelas absorbant ou "fluff". Une seconde incorporation de matière superabsorbante de quantité moindre est également effectuée mais cette fois ci plus en surface du fluff et, surtout, cette seconde incorporation est localisée dans une zone déterminée pour créer une région bénéficiant d'une absorption particulièrement adaptée. Ces couches culottes spécialement conçues pour les filles ou les garçons exploitent au mieux les propriétés des produits superabsorbants. Ces derniers sont incorporés dans les couches culottes uniquement dans les régions les plus exposées où ils se montrent le plus efficace et le plus rentable.

Pour la fabrication de ces couches culottes spécialisées, l'incorporation du produit superabsorbant s'opère durant la phase de formation du fluff dans le floculateur. Un premier canon à poudre projette la matière superabsorbante sur le fluff alors que celui-ci présente une épaisseur d'environ le tiers de celle définitive. Puis, un second canon à poudre projette de façon discontinue la matière superabsorbante pour créer des zones d'absorption privilégiées, ceci dans le fluff alors qu'il occupe une épaisseur d'environ les 2/3 de celle définitive.

L'inconvénient de cette technique est qu'il est très difficile d'aboutir à une délimitation géométrique rigoureuse de la zone d'absorption privilégiée. Le canon à poudre ne permet pas d'être suffisamment précis et la finesse du produit superabsorbant a tendance à augmenter la diffusion.

Le but principal de la présente invention est de présenter une couche culotte de type spécialisé qui présente un pouvoir d'absorption et de rétention des liquides encore accru et ceci de façon économique avec une utilisation égale sinon moindre de produit superabsorbant.

Ces avantages résultent d'une utilisation parfaitement contrôlée de produit superabsorbant. Dans la couche culotte de l'invention, une zone d'absorption privilégiée est créée avec une définition géométrique rigoureuse et une localisation du produit superabsorbant en surface du matelas absorbant.

Un autre but de la présente invention est de présenter un procédé de fabrication de couches culottes spécialisé selon lequel on maîtrise parfaitement l'incorporation de produit superabsorbant pour créer une zone d'absorption privilégiée ou la quantité et la délimitation de produits sont parfaitement maîtrisées. Il faut également souligner que la technique mise en oeuvre est parfaitement compatible avec les exigences industrielles de faisabilité et de productivité.

D'autres buts et avantages de la présente invention apparaîtront au cours de la description qui va suivre.

Selon l'invention, la couche culotte, qui trouvera notamment son application dans le domaine de l'hygiène infantile et/ou de l'incontinence, formée d'un film extérieur étanche, d'un matelas central de matériau absorbant éventuellement additionné de produit superabsorbant, d'un "tissue" et d'une feuille de confort, est caractérisée par le fait que du produit superabsorbant est fixé sur le tissue.

Le procédé de fabrication d'une couche culotte selon le paragraphe précédent par assemblage des différents éléments précités est caractérisé en ce qu'on fixe sur le "tissue" une certaine quantité de produit superabsorbant.

L'invention sera mieux comprise à la lecture de la description suivante, accompagnée de dessins en annexe parmi lesquels :
- la figure 1 montre en vue de coupe la structure interne d'une couche culotte,
- la figure 2 schématise une installation de production de fluff avec incorporation de produit superabsorbant,
- les figures 3 et 4 représentent à titre indicatif des bandes de matériau destinées à la fabrication de couches culottes spécialisées qui comprennent des zones d'absorption privilégiées respectivement soit pour filles soit pour garçons,
- la figure 5 représente une installation destinée à la mise en oeuvre du procédé de fabrication de la présente invention qui permet de fixer localement sur le tissue une quantité déterminée de produit superabsorbant.

La présente invention vise une couche culotte ainsi que son procédé de fabrication. Elle trouvera notamment son application dans le domaine de l'hygiène infantile et/ou médicale.

Depuis de nombreuses années, le change complet a supplanté le lange textile traditionnel. L'utilisateur a été très vite convaincu de ses avantages notamment sur le plan de la simplification d'utilisation conférée par les produits jetables. Le facteur économique étant néanmoins un point essentiel de la réussite, les fabricants se sont orientés vers l'élaboration de produits bon marché pour être compétitifs. Certains défauts inhérents à la simplicité de l'article ne pouvaient être solutionnés sans alourdir considérablement le coût de fabrication et il a fallu attendre de récents progrès technologiques pour parvenir à mettre au point une couche culotte dont la conception du produit permettait de commercialiser une couche culotte de bon rapport qualité/prix.

Les deux caractéristiques fondamentales qui ont été les clés du développement de ces couches furent le système de fermeture à adhésifs repositionnables et la mise au point de produits chimiques superabsorbants tels que le polyacrylate de sodium ou de potassium qui ont la propriété d'absorber et de retenir une quantité de liquide extrêmement importante de l'ordre de plusieurs dizaines de fois son propre poids.

Ce produit superabsorbant est toutefois très onéreux et une incorporation trop massive dans le matelas absorbant provoque un phénomène de "gel blocking" provoqué par la naissance d'une barrière gélatineuse s'opposant à la circulation des liquides.

Les plus récents développements de la technique ont abouti à l'élaboration de couches culottes spécialisées dans lesquelles une petite quantité de produit superabsorbant est mise en place dans une zone déterminée de la couche culotte pour créer une région à absorption privilégiée. L'efficacité du produit superabsorbant s'en trouve renforcée malheureusement les techniques actuelles de fabrication ne permettent pas de contrôler rigoureusement la géométrie de la région d'absorption privilégiée créée. Il s'en suit une efficacité qui n'est pas optimale et une partie du produit superabsorbant mis en jeu ne joue pas totalement son rôle.

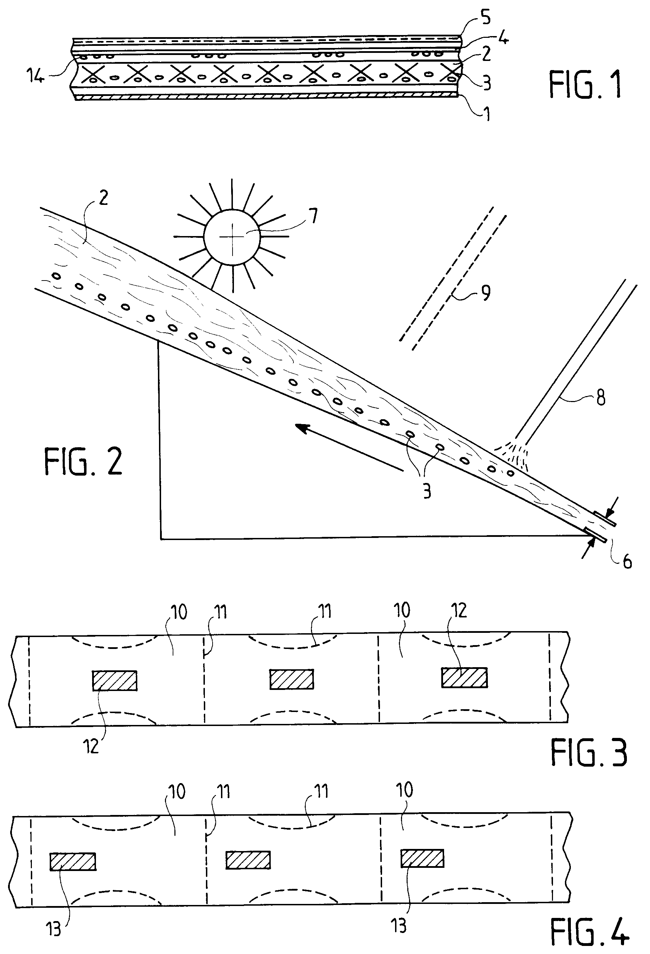

La figure 1 représente la structure interne d'une couche culotte. Elle comporte à la base un film 1 extérieur étanche au moins au liquide, généralement en polyéthylène, qui donne de la résistance à l'ensemble et qui évite les fuites. Ensuite, on trouve un matelas central 2 de matériau absorbant, tel qu'au moins à base de fibres cellulosiques, dont le rôle est l'absorption et la rétention de liquides.

Le matelas central 2 également appelé "fluff" doit, pour des raisons de confort, avoir une épaisseur modérée. Aussi, pour renforcer son pouvoir absorbant, on pourra avantageusement y inclure des produits chimiques superabsorbants qui se présentent sous la forme de poudres 3.

Le fluff 2 est recouvert d'un "tissue" 4, il s'agit d'une feuille de non-tissé dont le poids spécifique est généralement compris entre 17 et 19 g/m$^2$ par exemple. Le tissue 4 est lui-même recouvert d'une feuille de confort 5, en non-tissé, généralement constitué de fibres de polypropylène dont le poids spécifique est compris entre 17 et 23 g/m$^2$ par exemple.

La technique de fabrication de ce complexe est bien connue de l'homme de l'art. Généralement, les différents éléments se présentent à l'origine sous forme de bandes continues qui sont superposées dans des installations appropriées pour ensuite être fixées et découpées selon la forme des couches culottes.

La figure 2 montre une installation de formage de fluff 2 dans lequel on incorpore une quantité régulière de produit superabsorbant.

La matière qui sert à la confection du fluff est délivrée à l'entrée 6 de l'installation par un floculateur. Un cylindre denté 7 contrôle la sortie de la nappe 2 formée.

Pour incorporer dans le fluff 2 le produit superabsorbant 3, qui se présente sous forme pulvérulente, on utilise traditionnellement un canon 8 qui projette à la surface du fluff 2 en formation la fine poudre de produit superabsorbant.

Le débit du canon 8 est contrôlé et généralement on le place de telle sorte que le produit superabsorbant 3 soit déposé à la surface du fluff 2 alors que celui-ci occupe sensiblement le tiers de son épaisseur définitive. Ceci afin que le produit superabsorbant 3 soit situé sensiblement au tiers de l'épaisseur du fluff 2 en sortie d'installation.

En pointillé est représenté un second canon à poudre 9 utilisé dans les installations connues traditionnelles de fabrication de couches culottes spécialisées. Le rôle de ce second canon 9 est de créer dans le fluff des zones d'absorption privilégiées.

Pour cela, à intervalles réguliers correspondant au pas de fabrication des couches culottes, le canon 9 envoie une quantité prédéterminée de produit superabsorbant qui s'étale à la surface du fluff en formation et crée ainsi une région dotée d'un pouvoir absorbant renforcé.

Il est nécessaire de contrôler avec précision la zone de projection de cette seconde émission de produit superabsorbant pour exploiter au maximum les caractéristiques du produit chimique. Cette zone d'absorption privilégiée, placée plus en surface du fluff 2 a une action plus immédiate et favorise l'assèchement en surface de la couche culotte. Par ailleurs, cette zone à absorption privilégiée améliore la garde au sec et l'humidité de surface.

Les figures 3 et 4 montrent respectivement les bandes continues de complexe 10, destinées à être découpées selon les pointillés 11 pour former des couches culottes, et qui possèdent chacune des zones d'absorption privilégiées respectivement 12 et 13 dont la localisation par rapport à la découpe de la couche culotte est déterminée en fonction de l'anatomie du bébé soit pour filles à la figure 3 et pour garçons à la figure 4.

Dans la pratique, il s'avère que le canon 9 à poudre est plus ou moins bien adapté pour limiter l'éjection de poudre de produit superabsorbant selon une géométrie bien déterminée. Des analyses montrent que l'on assiste plutôt à la création d'une zone d'aspersion au contour flou.

La présente invention se propose précisément d'apporter une solution à cet inconvénient. En particulier, le produit superabsorbant 14 destiné à créer une zone d'absorption privilégiée est fixé sur le tissue 4 tel qu'illustré à la figure 1.

De nombreux avantages en découlent. Tout d'abord, puisqu'il y a fixation directe, les risques de migration ultérieurs de la poudre de produit superabsorbant sont totalement écartés. Par ailleurs, le produit 14 superabsorbant qui est placé en surface du fluff 2 exerce une action immédiate d'absorption des liquides et assèche très rapidement la surface interne de la couche culotte, d'où un confort accru. La diffusion de la masse liquide au sein du matériau absorbant 2 est également amélioré.

Cela étant, il est avantageux que le produit superabsorbant 14 soit fixé sur la face interne du tissue 4, c'est-à-dire du côté matelas absorbant 2, afin d'éloigner la poudre de la peau de l'enfant qui risquerait d'être irritée en cas de contact.

Le produit superabsorbant 14 est fixé localement sur la face interne du tissue 4 pour former des zones privilégiées à haut pouvoir absorbant. Selon l'invention, la géométrie des zones privilégiées peut être parfaitement définie grâce à une détermination exacte des zones de fixation.

De la sorte, il est possible d'utiliser une très faible quantité de produit superabsorbant tout en bénéficiant d'une action très importante. Des essais ont permis de déterminer qu'avec 1,5 g de produit superabsorbant, on pouvait créer une région rectangulaire d'absorption privilégiée 12 ou 13 dont les dimensions sont 100 mm x 190 mm par exemple.

La fabrication d'une telle couche culotte reste compatible avec les techniques actuelles d'assemblage en bandes des différents composants pour former un complexe si ce n'est que le tissue doit recevoir le produit superabsorbant avant l'assemblage.

Ainsi, selon le procédé de fabrication de la présente invention, on fixe sur la bande de tissue une certaine quantité de produit superabsorbant. Grâce à une parfaite maîtrise de la technique de fixation, il est possible de définir des zones géométriques précises renfermant des quantités déterminées de produit superabsorbant.

Pour cela, on crée sur le tissue une ou plusieurs zones dotées de pouvoir de fixation et on projette sur le tissue une quantité prédéterminée de produit superabsorbant.

En pratique, on encolle le tissue dans les zones privilégiées pour créer des régions correspondantes adhésives, puis on projette sur le tissue encollé en défilement un débit déterminé de produit superabsorbant, et ensuite on récupère le produit superabsorbant non fixé. Ainsi, seules les zones encollées du tissue sont enduites de produit superabsorbant et ceci avec une quantité prédéterminée.

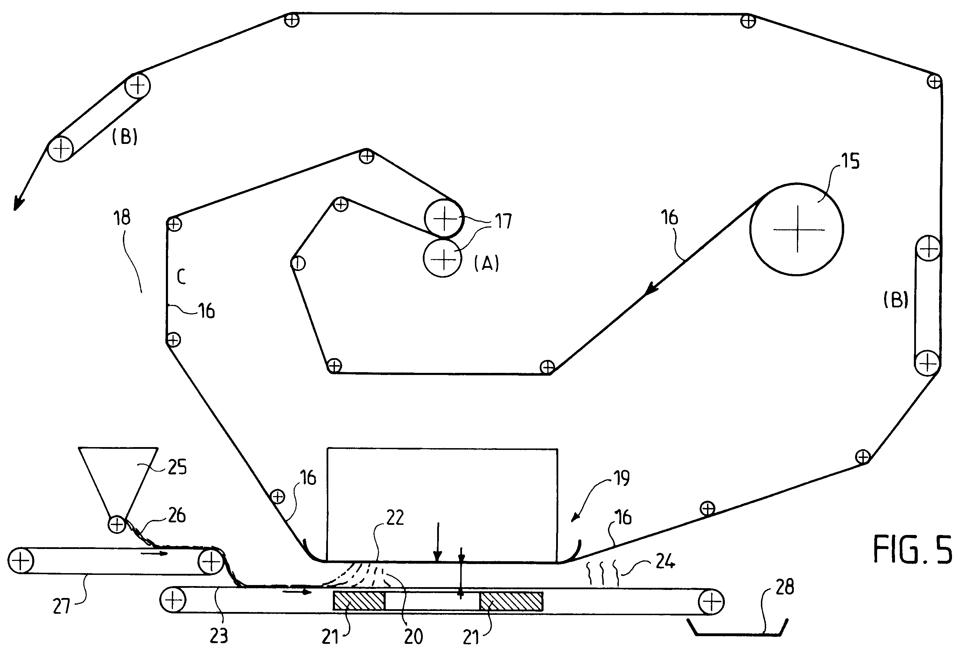

Pour la mise en oeuvre du procédé de l'invention, on pourra par exemple utiliser une installation telle qu'illustrée à la figure 5. Elle comporte tout d'abord un dévidoir 15 sur lequel le tissue est enroulé et dont le rôle est de le délivrer sous forme de bande continue 16. Des cylindres 17 motorisés assurent le défilement régulier de la bande 16 sous tension constante.

L'installation comporte ensuite un poste d'encollage 18 de la bande de tissue 16. Les techniques d'encollage qui peuvent être utilisées sont à la portée de l'homme de l'art. A titre d'exemple, une rampe à lèvre peut être utilisée pour déposer une couche de colle par contact, ou un cylindre lécheur qui comporte à sa surface une empreinte correspondant à la forme géométrique de la surface à haut pouvoir absorbant qui doit être créée pourra également être utilisé.

L'avantage de cette dernière technique est qu'il est possible de procéder à une enduction de colle discontinue notamment par points sur la surface de la bande de tissue. On pourra également procéder par pulvérisation de colle à travers des buses avec l'emploi conjoint d'un masque pour assurer le contrôle de la surface enduite.

On trouve ensuite dans l'installation le poste 19 de fixation du produit superabsorbant sur la bande 16 de tissue. Selon un mode de réalisation du procédé de l'invention, on fait défiler la bande 16 de tissue dans le poste 19 avec la face encollée dirigée vers le bas. De la sorte, quoique l'on projette le produit superabsorbant 20 sur la totalité de la surface inférieure de la bande 16 de tissue, seules les zones encollées ont le pouvoir de retenir la poudre de produit absorbant. Le reste de la surface de la bande 16 de tissue n'a pas la capacité pour retenir des particules de produit superabsorbant et par gravité celles-ci retombent et sont récupérées dans l'installation.

On peut envisager d'utiliser plusieurs dispositifs à même de projeter régulièrement une certaine quantité de produit superabsorbant sur la face inférieure par exemple au moyen d'un accélérateur de particules.

Cet accélérateur se compose d'une ou plusieurs électrodes haute tension inférieures 21 et électrodes 22 supérieures entre lesquelles on fait défiler la bande de tissue 16 telle qu'illustrée à la figure 5. Entre les électrodes 21 et 22, on crée un champ électrique important, généralement de l'ordre de 8.000 volts pour un entrefer de 70 à 80 mm.

Les particules de produit superabsorbant renferment naturellement dès l'origine des particules d'eau. Cette humidité résiduelle a pour effet de permettre la polarisation des particules de poudre de matière superabsorbante qui dès lors se voit attirée d'une électrode 21 à l'autre 22.

On assiste dans l'entrefer à une projection des particules de poudre de produit superabsorbant qui viennent se plaquer contre la face inférieure de la bande de tissue dont certaines zones sont adhésives. Ces zones retiennent les particules qui se trouvent définitivement fixées sur la bande de tissue 16. Une fois celle-ci hors de l'accélérateur de particules 19, le champ électrique s'estompe et les particules non fixées sur la bande de tissue 16 retombent par gravité et sont récupérées.

Pour assurer une enduction de quantité déterminée de produit superabsorbant, on contrôle la quantité de produit superabsorbant 20 placé dans l'entrefer de l'accélérateur de particules 19.

A cet égard, l'invention préconise par exemple l'emploi d'un convoyeur linéaire à bande 23 dont le tapis est en défilement continu dans l'entrefer de l'accélérateur 19. On dépose à la surface du tapis du convoyeur 23 en amont de l'accélérateur de particules 19 une quantité régulière de produit superabsorbant. Ainsi, on alimente avec un débit constant un produit superabsorbant, l'accélérateur de particules 19.

La quantité de poudre 20 projetée sur la face interne de la bande de tissue 16 est régulière et c'est seulement en sortie d'accélérateur de particules 19 que l'on récupère l'excédent de produit superabsorbant précédemment projeté. Le convoyeur linéaire 23 s'étend au-delà de l'accélérateur de particules 19 et par conséquent on récupère à sa surface l'excédent 24 de produit superabsorbant par exemple en 28 l'extrémité du convoyeur linéaire 23.

Pour alimenter le convoyeur linéaire 23 régulièrement en produit superabsorbant, selon l'invention, on peut utiliser une trémie 25 qui contient le produit superabsorbant et dont on peut régler la quantité de produit délivré en sortie 26. La trémie 25 délivre du produit 26 sur un convoyeur linéaire auxiliaire 27 qui a la particularité d'être peseur.

De la sorte, on peut régulièrement disposer à sa surface une quantité régulière de produit superabsorbant. Le tablier du convoyeur auxiliaire 27 est animé d'un mouvement de translation uniforme et déverse le produit superabsorbant à la surface du convoyeur linéaire 23 comme cela est schématisé à la figure 5. En ajustant le débit de sortie de la trémie 25, en fonction du poids mesuré à la surface du convoyeur auxiliaire 27, il est possible d'assurer un débit de produit superabsorbant constant à l'entrée de l'accélérateur de particules 19.

Quoique différentes techniques de fixation du produit superabsorbant puissent être utilisées, des essais concluant ont été menés avec l'installation telle qu'illustrée à la figure 5 en utilisant pour le collage un hot-melt appliqué sur le tissue à raison de 2 g/m$^2$ par exemple. La vitesse de défilement du tissue était comprise entre 30 et 150 m/mn.

Bien entendu, l'installation d'encollage et de fixation du produit superabsorbant sur le tissue est synchronisée avec la ligne de production des couches culottes et en particulier l'unité de découpage afin de positionner avec précision la région à absorption privilégiée dans la couche culotte produite.

D'autres mises en oeuvre de la présente invention à la portée de l'homme de l'art auraient également pu être envisagées sans pour autant sortir du cadre de celle-ci.

**Revendications**

1. Couche culotte, qui trouvera notamment son application dans le domaine de l'hygiène infantile et/ou médicale, formée d'un film extérieur étanche (1), d'un matelas central de matériau absorbant (2) éventuellement additionné de produit superabsorbant (3), d'un tissue (4) et d'une feuille de confort (5), caractérisée par le fait que du produit superabsorbant (14) est fixé sur le tissue (4).

2. Couche culotte selon la revendication 1, caractérisée par le fait que du produit superabsorbant (14) est fixé sur la face interne du tissue (4).

3. Couche culotte selon la revendication 1, caractérisée par le fait que du produit superabsorbant (14) est fixé localement sur la face interne du tissue (4) pour former des zones privilégiées à haut pouvoir absorbant (12, 13).

4. Couche culotte selon la revendication 1, caractérisée par le fait que du produit superabsorbant (14) est fixé par collage sur le tissue (4).

5. Couche culotte selon la revendication (3), caractérisée par le fait que les zones privilégiées (12, 13) à haut pouvoir absorbant renferment environ 1,5 g de produit superabsorbant pour une surface rectangulaire d'environ 100 x 190 mm.

6. Procédé de fabrication d'une couche culotte, selon la revendication 1, par assemblage d'un film extérieur étanche (1), d'un matelas central (2) de matériau absorbant éventuellement additionné de produit superabsorbant (3), d'un tissue (4) et d'une feuille de confort (5), caractérisé en ce que l'on fixe sur le tissue (4) du produit superabsorbant (14).

7. Procédé de fabrication d'une couche culotte selon la revendication 6, caractérisé en ce que :
   - on crée sur le tissue (4) une ou plusieurs zones (12, 13) dotées de pouvoir de fixation,
   - on projette sur le tissue (4) une quantité prédéterminée de produit superabsorbant (14).

8. Procédé de fabrication d'une couche culotte selon la revendication 7, caractérisé en ce que :
   - on encolle le tissue (16) pour créer une ou plusieurs zones adhésives,
   - on projette sur le tissue (16) en défilement un débit déterminé de produit superabsorbant (20),
   - on récupère le produit superabsorbant (24) non fixé sur la bande de tissue (16).

9. Procédé de fabrication d'une couche culotte selon la revendication 8, caractérisé en ce que :
   - on encolle des zones privilégiées à haut pouvoir absorbant sur la bande (16) de tissue,
   - on fait défiler la bande de tissue (16) avec la face encollée dirigée vers le bas dans un accélérateur (19) de particules alimentées à débit constant dans l'entrefer en produit superabsorbant.

10. Procédé de fabrication d'une couche culotte selon la revendication 9, caractérisé en ce que :
    - on encolle des zones privilégiées sur la bande de tissue (4),
    - on fait défiler la bande de tissue avec la face encollée dirigée vers le bas dans un accélérateur de particules (19) dont l'entrefer est traversé par un convoyeur linéaire (23) dont le tapis est chargé régulièrement en produit superabsorbant.

FIG. 1

FIG. 2

FIG. 3

FIG. 4

FIG.5

European Patent Office

# EUROPEAN SEARCH REPORT

Application Number

EP    91 44 0014

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | US-A-4 715 918 ( KIMBERLY-CLARK CORPORATION ) <br> * figures 1,6-8,12 * <br> * column 1, line 65 - column 2, line 7 * <br> * column 2, line 43 - column 3, line 42 * <br> --- | 1,4,6-10 | A61F13/15 |
| X | EP-A-0 374 105 ( FARICERCA S. P. A. ) <br> * figures 2,3 * <br> * abstract * <br> * page 3, line 46 - line 51 * <br> * page 4, line 5 - line 48 * <br> ----- | 1-3,6 | |
| | | | TECHNICAL FIELDS SEARCHED (Int. Cl.5) |
| | | | A61F |

The present search report has been drawn up for all claims

| Place of search | Date of completion of the search | Examiner |
|---|---|---|
| THE HAGUE | 25 OCTOBER 1991 | ARGENTINI A. |